# EUROPEAN PATENT SPECIFICATION

(11) **EP 2 170 348 B1**
(45) Date of publication and mention of the grant of the patent: **10.08.2016**
(21) Application number: 08784529.3
(22) Date of filing: 20.06.2008
(51) Int. Cl.: A61K 31/573, A61K 9/46, A61K 9/72

(54) **EFFERVESCENT TABLETS FOR INHALATORY USE**
BRAUSETABLETTEN ZUR INHALATION
COMPRIMÉS EFFERVESCENTS DESTINÉS À ÊTRE INHALÉS

(30) Priority: 22.06.2007 EP 07110896
(43) Date of publication of application: 07.04.2010
(73) Proprietor: Dompé farmaceutici s.p.a., 20122 Milano (IT)
(72) Inventor: GENTILE, Marco, I-67100 L'Aquila (AQ) (IT); CANTARINI, Marco, I-67100 L'Aquila (AQ) (IT)
(74) Representative: Pistolesi, Roberto
(86) International application number: PCT/EP2008/004993
(87) International publication number: WO 2009/000473

(56) References cited:
- WO-A-03/035030
- WO-A-03/086347
- WO-A-2004/022132
- WO-A-2005/011639

## Description

### Brief description of the invention

The present invention relates to effervescent tablets containing at least one micronized water-insoluble active principle for the extemporaneous preparation of a suspension suitable for inhalatory use.

Furthermore, it relates to the use of effervescent tablets containing water-insoluble active principles for the extemporaneous preparation of a suspension for inhalatory use.

The tablets are added to water or to a solution wherein they disaggregate until a fine suspension is obtained. The suspension maintains the initial particle size distribution of the active principle, ensuring the achievement of the therapeutic window of the breathed fraction.

### Background of invention

Particle size is a very important factor to consider when administering inhalation preparations. It has been reported that the optimum particle size for penetration into the pulmonary cavity falls in the range 0.5 - 7.0 µm.

Smaller particles fail to settle in the bronchioles and bronchi and are mainly exhaled, while bigger particles get blocked in the respiratory tree before entering the bronchioles.

Water-insoluble drugs such as corticosteroids and mucolytic agents are often used by inhalatory administration in the form of suspensions for nebulisation, generally from a metered aerosol. This way of administration has the advantage that the drug exerts a topical effect on the lungs without a significant systemic activity at recommended doses.

For example, cortiscosteroids such as beclomethasone dipropionate, beclomethasone dipropionate, fluticasone propionate, budesonide, flunisolide, betamethasone, triamcinolone, mometasone, ciclesonide are widely used for the prophylaxis of the symptoms of asthma by inhalation.

However, commercial products of corticosteroid active principles, such as beclomethasone inhalatory suspensions, show the inconvenience of a very variable particle size distribution due to the formation of aggregates, this affecting the fraction of drug that actually reaches the lower parts of the respiratory tract. Furthermore, the formation of aggregates causes the sedimentation of the particles of active principle, thereby affecting also the dosing of the active principle.

WO 2004/022132 A2 discloses an insoluble carrier impregnated with the active substance in micronized form which is reconstituted prior to administration by addition of a solution for inhalation.

### Summary of the Invention

The present invention is defined in the claims. The present inventors have surprisingly found that the problem of the wide granulometric dispersion of commonly used inhalatory suspensions can be overcome by the extemporaneous preparation of inhalatory suspension from effervescent tablets. In fact, as it will be shown in details in the experimental section hereibelow, it has now been found that suspensions made from effervescent tablets retain the original particle size of the drug substance.

Thus, object of the present invention are effervescent tablets comprising a micronised water insoluble active ingredient and their use for the preparation of a suspension for inhalatory administration.

The use of the effervescent tablets of the invention allows to obtain a suspension that retain the original particle size of the drug substance and, giving better particle size patterns, enhances the efficacy of the topical treatment..

The suspension, extemporarily reconstituted introducing an effervescent tablet into a nebulization device previously filled with an aqueous solution, preferably a physiological solution, is inhaled after the complete disintegration of the tablet, providing a composition that falls into the therapeutical particle size range.

There are other advantages in using a solid formulation instead of a solution or a suspension; solid drug products in fact have a better chemical and physical stability, and a easier use and shipping.

Moreover, they do not need preservatives in the formulation, this raising the acceptability and tolerability of the administered product.

When the half dose is needed, tablets can be divided in two parts, and the stability of the residual half tablet is far bigger than the half dose of a solution or suspension.

### Brief description of the figures

The specification is accompanied by the following figures:
**Figures 1A** **and** **1B** show a comparison between the particle size distribution of beclometasone dipropionate (BDP) raw material and suspension from effervescent tablet;
**Figures 2A** **and** **2B** show a comparison between the particle size distribution of beclometasone dipropionate (BDP) raw material and commercially available BDP aqueous suspension;
**Figure 3A** **and** **3B** show a comparison between the particle size distribution of fluticasone propionate raw material and suspension from effervescent tablet.

### Detailed description of the invention

A first object of the invention is an effervescent tablet for the extemporaneous preparation of a suspension for inhalatory administration comprising a micronized water-insoluble active principle.

The active principle present in the tablet of the present invention has been micronized before formulation into the tablet to a particle size between 0.5 and 7 µm.

According to the present invention, the term "water-insoluble active principle" indicates that the solubility of the active principle is such that from 100 mL to more than 10,000 mL of water are necessary to dissolve 1 gram of molecule

Preferred active principles for use in the tablet of the present invention are corticosteroids such as beclometasone dipropionate, fluticasone propionate, budesonide, flunisolide, betamethasone, triamcinolone, mometasone, ciclesonide or water-insoluble mucolytic agents such as sobrerol. These active principles can be used alone or in combination.

Most preferred compounds that can be vehicled the effervescent tablets are beclometasone dipropionate and fluticasone propionate.

The composition of excipients used in the effervescent tablet must be such that the tablet dissolves in short time, with a quick effervescence that does not leave remainders or slugs not completely dissolved.

The excipients of the composition have to be compatible with the administration route at issue (cfr. Inactive Ingredient Guide, FDA, 1996 - Inhalatory route).

In details the tablet of the invention comprises the following excipients:
Acidic compounds: citric acid (anhydrous, or monohydrate, or dihydrate), tartaric acid. Basic compounds: carbonate, bicarbonate, baked carbonate, or baked bicarbonate.
Surfactants: they are responsible for the wetting of the poor soluble drug substance in the aqueous medium, after the complete dissolution of the tablet. Non-ionic surfactants (e.g. polysorbates) as well as ionic surfactants (e.g. SDS) can be used.

Other optional excipients are the following:
Binders: if the wet granulation is chosen, a binding solution is necessary to form the granules. PVP and other common binders can be used, depending on the technique chosen.
Adsorbents: can be useful to adsorb the overwetting of granules.
Lubricants: they have to be compatible with the inhalation route. Sodium benzoate or similar substances can be effective in lubricating the mixtures before pressing.
Diluents: it is necessary to reach a suitable final weight of the tablet. Lactose, mannitol, starch and other diluents can be used for this purpose.

The excipients do not have to aggravate the overall flavor and odor of the tablet; in some cases they have to correct some unfavourable organoleptic properties of the drug substance.

For this purpose, a taste modifying agent could be necessary to mask the bad taste of the drugs. It has to be reminded, in fact, that the steam supplied by the nebulizer condense in the throat or in the nose, thus promoting the detection of bitterness, sourness ands so on.

Methods of production of effervescent tablets can be different, according to special needs of the drug substance that has to be formulated.

Production can occur by direct compression of drug substance and excipients or after a granulation step.

Drug substances can be either granulated with the excipients or added as powders to the granulated excipients, but tests showed that the best particle size profiles were obtained by granulating the drug substance with at least some of the excipients.

### Examples of granulation techniques are the following:

### 1a. Wet granulation/fluid bed granulation

Usually, there is an acidic and a basic carbonic compound in the mixture. These excipients are responsible for the effervescence of the tablets, when they are placed in an aqueous environment. When choosing a wet or fluidized bed granulation technique, it is important to keep the acidic and the basic compound well separated (e.g. the acidic compound is granulated with the drug substance and/or some other excipients, while the basic compound is added to the dried granules, before the tablet pressing), in order to avoid the onset of effervescence process during the production.

### Examples of wet granulation / fluidized bed granulations are the following:

### a. Heated mixing on an oil bath or a sand heater.

Powders are mixed under heat. When excipients containing bound molecules of water are used, the water acts as a granulating agent in the bulk, while freeing itself from the crystalline structure. The mixing step aids the distribution of the water, thus providing an homogenous mixture of wet granules.

The granules are then sieved and dried until the desired water content.

### b. Fluid bed granulation.

Part of the bulk (excipients and/or the drug substance) is granulated with water and a binding solution (e.g. polyvinylpyrrolidone). The granules are then dried in the fluid bed until the desired residual humidity is reached.

### 1b. Dry granulation

Dry powders are mixed and pressed in big slugs or ribbons. These agglomerates are then crushed into smaller granules until the desired particle size.

The original powder mixture of drug substance and excipients has to be compressible and flowing to be easily compressed. It should not tend to pack in the feeders and the tooling of the tablet press, as well.

A further object of the present invention is the use of the effervescent tablet of the invention for the extemporaneous preparation of a suspension for inhalatory administration.

The suspension is prepared adding the effervescent tablet in water or in an aqueous solution, where it disintegrates. Preferably, said water or aqueous solution is sterile.

According to a preferred embodiment of the invention the aqueous solution is a physiological solution.

Tablets can alternatively be added also to a solution of at least one water-soluble active principle. Preferably said active principles are water-soluble mucolytic agents, preferably selected from the group consisting of ambroxol or sobrerol, or bronchodilators, preferably selected from salbutamol or ipratropium chloride.

### Examples

### Beclometasone dipropionate effervescent tablets.

Preparation of an effervescent tablet containing 0.8 mg of beclometasone dipropionate. The composition of the tablet prepared for the present study is reported in table 1.

**Table 1 - composition of beclometasone dipropionate effervescent tablets.**

| ***Ingredient*** | ***Amount per tablet*** |
|---|---|
| Beclometasone dipropionate | 0.8 mg |
| Sodium bicarbonate | 31 mg |
| Citric acid anhydrous | 25 mg |
| Lactose | 8.4 mg |
| Polysorbate 20 | 2 mg |
| Sodium benzoate | 2.8 mg |

The above ingredients are mixed using the fluid bed granulation technique above described and granules are then pressed.

Once the tablet is put in the inhalation device vessel, previously filled with physiological solution, a suspension forms after the fast disintegration of the tablet. The technological properties of the tablet and the reconstituted suspension after disintegration are summarized in table 2.

**Table 2 - properties of the tablet / reconstituted suspension.**

| ***Tablet*** | |
|---|---|
| Mean weight | 70 mg |
| Diameter | 5 mm |
| Thickness | 2.0 - 2.2 mm |
| Water content | 0.2 - 0.3% |
| Mean crushing strength | 18 N |
| Colour | White |
| Mean disintegration time | 150 seconds |

| ***Suspension*** | |
|---|---|
| pH | 5.0-7.0 |
| Colour | White |
| Opalescence | Yes |
| Residue or precipitate presence | No |
| Foaminess | Yes |

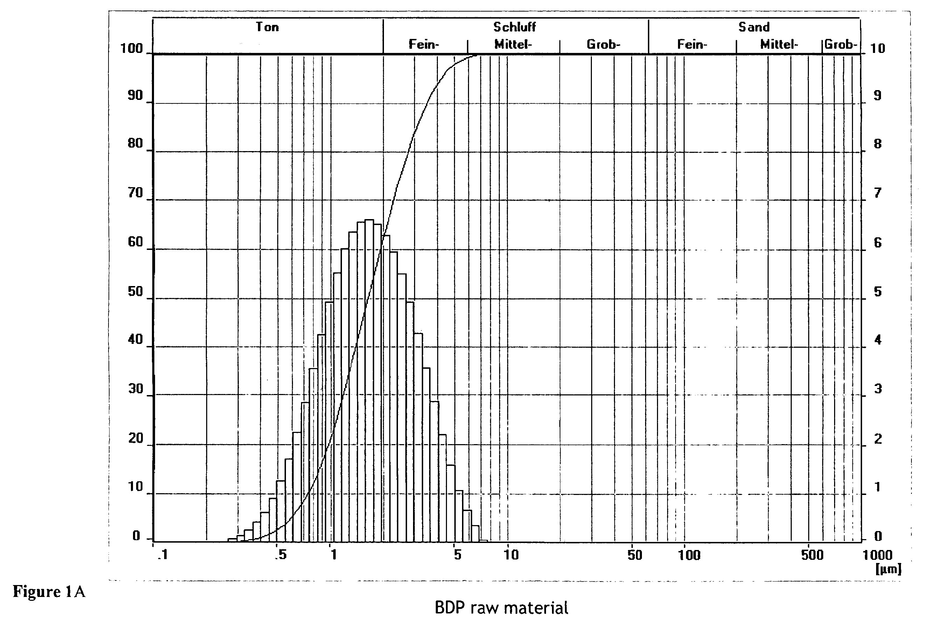

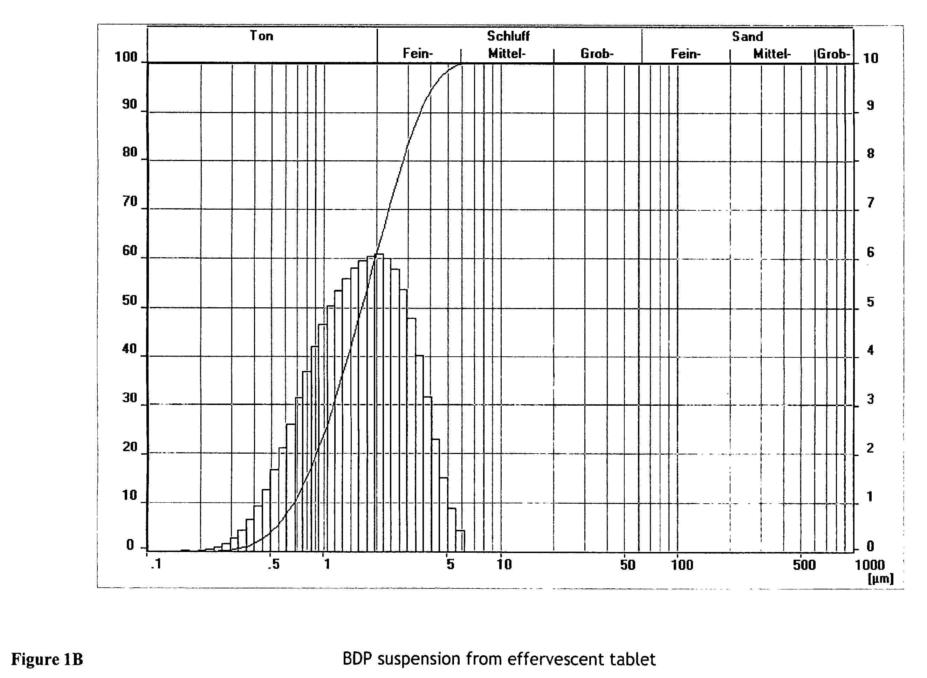

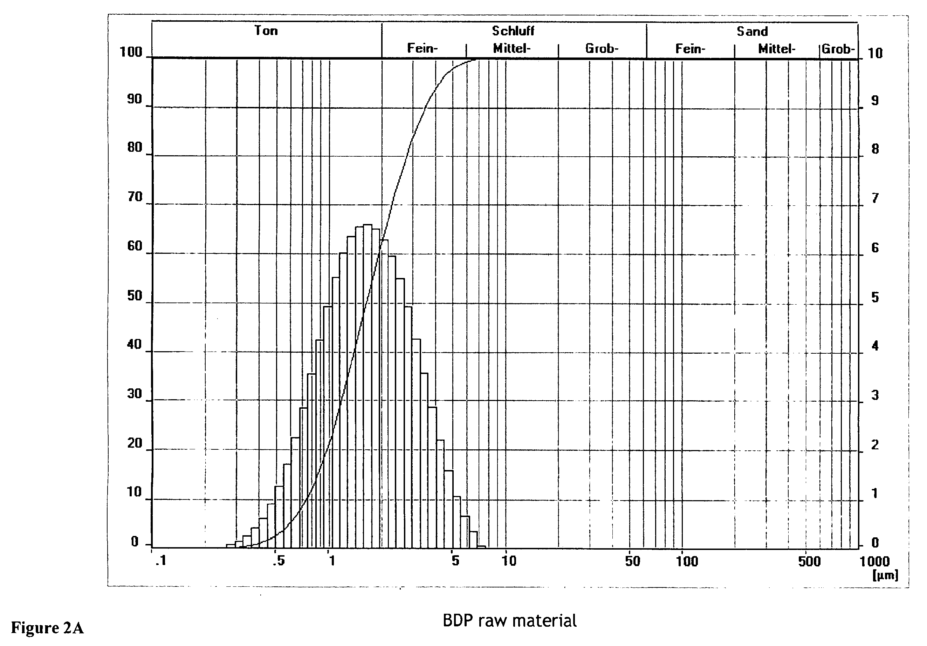

A granulometric analysis was performed on the final suspension. The distribution curve was compared with the commercial raw material (material which is used for the preparation of commercial available BDP). As it can be seen from data displayed in table 3 and figures 1A-1B and 2A-2B, there is little or no difference between the particle size of the raw material and the suspension obtained by disintegration of the effervescent tablet.

**Table 3 - granulometric analysis of beclometasone dipropionate of raw material and suspension from effervescent tablet.**

| ***Raw material*** | |
|---|---|
| Median | 1.638 µm |
| Mode | 1.654 µm |
| 100% of particles | < 7.62 µm |

| ***Suspension from effervescent tablet*** | |
|---|---|
| Median | 1.642 µm |
| Mode | 2.031 µm |
| 100% of particles | < 6.86 µm |

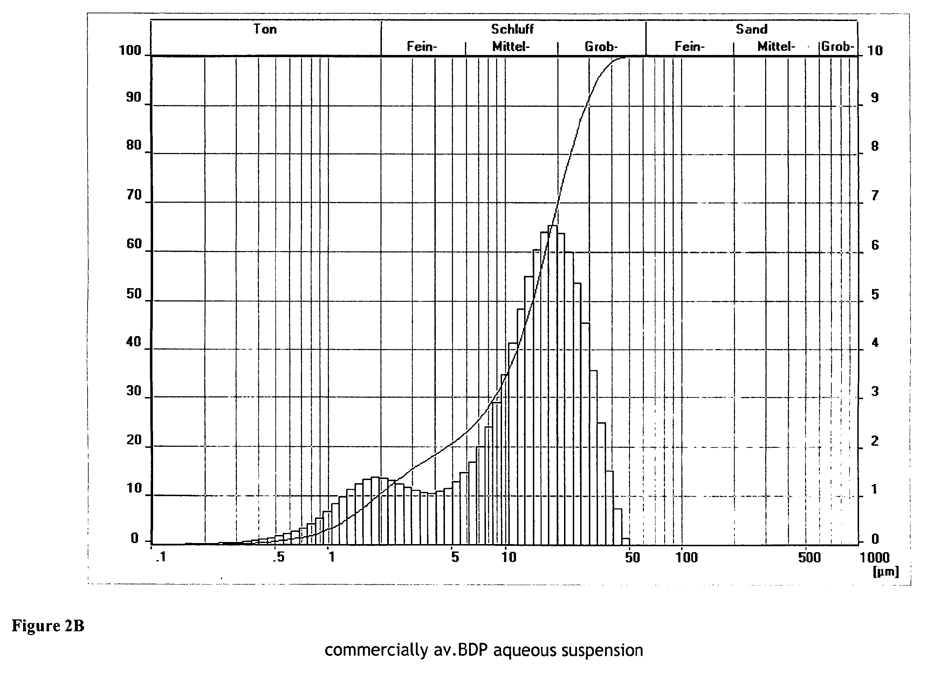

As a proof of concept, here is reported the granulometric analysis and particle size distribution of a commercially available aqueous BDP inhalatory suspension. As it can be seen from table 4 and figures 2A and 2B, the particle size of the aqueous suspension is broader, and the mean values are considerably bigger than the raw material.

**Table 4 - granulometric analysis of beclometasone dipropionate in a commercially available aqueous BDP inhalatory suspension.**

| ***Aqueous inhalatory suspension*** | |
|---|---|
| Median | 14.239 µm |
| Mode | 18.484 µm |
| 100% of particles | < 50.47 µm |

### Fluticasone propionate effervescent tablets.

Preparation of an effervescent tablet containing 0.5 mg of fluticasone propionate. The composition of the tablet prepared for the present study is reported in table 5.

**Table 5 - composition of fluticasone propionate effervescent tablets.**

| ***Ingredient*** | ***Amount per tablet*** |
|---|---|
| Fluticasone propionate | 0.5 mg |
| Sodium bicarbonate | 22.15 mg |
| Citric acid anhydrous | 18.4 mg |
| Lactose | 26.315 mg |
| Polysorbate 20 | 0.085 mg |
| Sodium benzoate | 2.55 mg |

The above ingredients are mixed using the fluid bed granulation technique above described and granules are then pressed.

Once the tablet is put in the inhalation device vessel, previously filled with physiological solution, a suspension forms after the fast disintegration of the tablet. The technological properties of the tablet and the reconstituted suspension after disintegration are summarized in table 6.

**Table 6 - properties of the tablet / reconstituted suspension.**

| ***Tablet*** | |
|---|---|
| Mean weight | 70 mg |
| Diameter | 5 mm |
| Thickness | 2.0 - 2.2 mm |
| Water content | 0.2 - 0.3% |
| Mean crushing strength | 28 N |
| Colour | White |
| Mean disintegration time | 60 seconds |

| ***Suspension*** | |
|---|---|
| pH | 5.0 - 7.0 |
| Colour | White |
| Opalescence | Yes |
| Residue or precipitate presence | No |
| Foaminess | Yes |

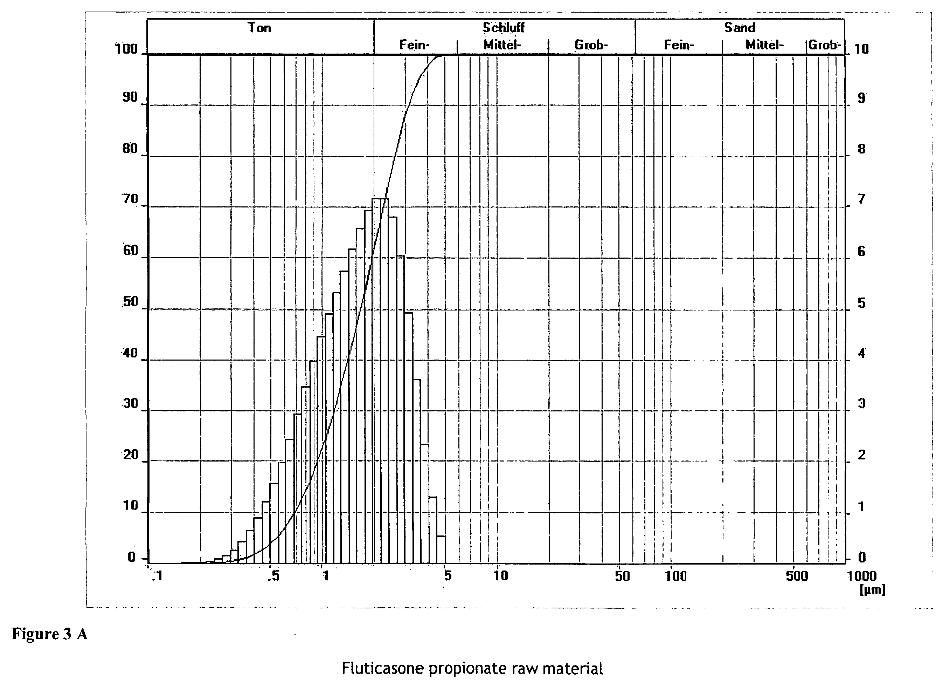

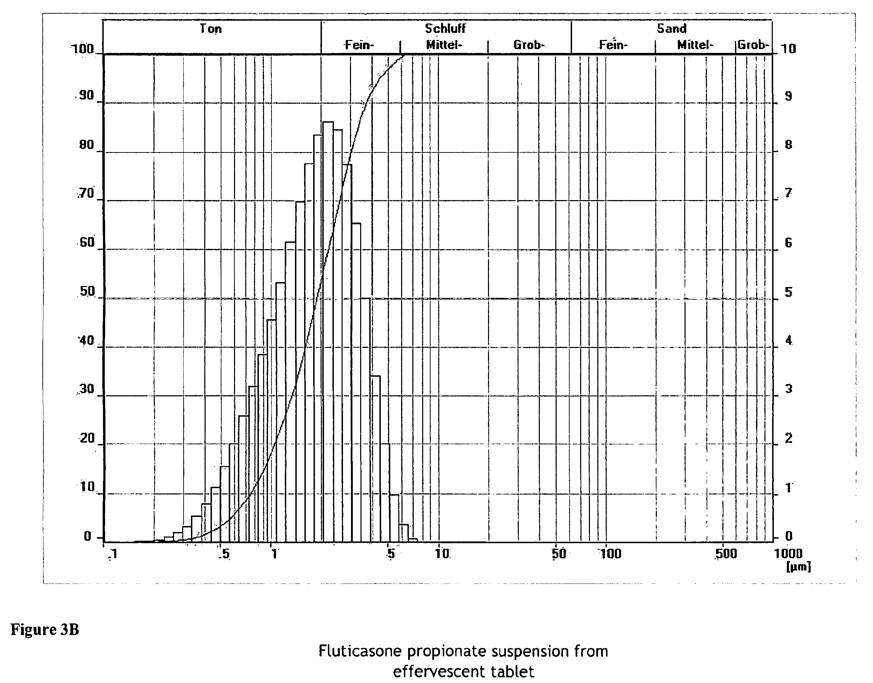

A granulometric analysis was performed on the final suspension. The distribution curve was compared with the commercial raw material (material which is used for the preparation of commercial available BDP). As it can be seen from data displayed in table 7 and figures 3A and 3B, there is little or no difference between the particle size of the raw material and the suspension obtained by disintegration of the effervescent tablet.

**Table 7 - granulometric analysis of fluticasone propionate of raw material and suspension from effervescent tablet.**

| ***Raw material*** | |
|---|---|
| Median | 1.664 µm |
| Mode | 2.135 µm |
| 100% of particles | < 5 µm |

| ***Suspension from effervescent tablet*** | |
|---|---|
| Median | 1.89 µm |
| Mode | 2.187 µm |
| 100% of particles | < 7.5 µm |

## Claims

1. Effervescent tablet for the extemporaneous preparation of a suspension suitable for inhalatory administration comprising at least one water-insoluble active principle and excipients suitable for inhalatory administration comprising:
an acidic compound selected from citric acid, tartaric acid,
a basic compound selected from carbonate, bicarbonate, baked carbonate and baked bicarbonate; non-ionic or ionic surfactants,
wherein said water-insoluble active principle has been micronized to a particle size between 0.5 to 7 µm and
wherein said effervescent tablet, once added to water or to an aqueous solution, disaggregates until obtaining said extemporaneous suspension suitable for inhalatory administration, in which the original particle size distribution of said water insoluble active principle is maintained.

2. Effervescent tablet according to claim 1, wherein said active principle is selected from corticosteroids and mucolytic agents.

3. Effervescent tablet according to claims 1 or 2, wherein said active principle is selected from the group consisting of beclomethasonedipropionate, fluticasone propionate, budesonide, flunisolide, betamethasone, triamcinolone, mometasone, ciclesonide and sobrerol.

4. Effervescent tablet according to claims 1 to 3 wherein said active principle is selected from beclomethasonedipropionate and fluticasone propionate.

5. Use of an effervescent tablet according to claims 1 to 4 for the preparation of an extemporaneous suspension for inhalatory administration.

6. Use according to claim 5, comprising the step of disintegration of the effervescent tablet in water or in an aqueous solution.

7. Use according to claim 6, wherein said aqueous solution is a physiological solution.

8. Use according to claims 6 to 7, wherein said aqueous solution is a solution of at least one water-soluble active principle.

9. Use according to claim 8, wherein said water-soluble active principle is a mucolytic agent or a bronchodilator.

10. Use according to claim 9, wherein said mucolytic agent is selected from the group consisting of ambroxol or sobrerol.

11. Use according to claim 9, wherein said bronchodilator is salbutamol or ipratropium chloride.

## Patentansprüche

1. Brausetablette zur unvorbereiteten Zubereitung einer Suspension, die zur Verabreichung mittels Inhalation geeignet ist und mindestens ein Wasser-unlösliches aktives Prinzip und Exzipienten umfasst, die zur Verabreichung mittels Inhalation geeignet sind, umfassend:
eine saure Verbindung ausgewählt unter Zitronensäure, Weinsäure,
eine basische Verbindung ausgewählt unter Carbonat, Bicarbonat, gebackenem Carbonat und gebackenem Bicarbonat;
nicht-ionischen oder ionischen oberflächenaktiven Mitteln,
worin das Wasser-unlösliche aktive Prinzip auf eine Teilchengröße zwischen 0,5 bis to 7 µm zerkleinert wurde, und
worin sich die Brausetablette nach Zugabe zu Wasser oder zu einer wäßrigen Lösung zersetzt, bis die zur Verabreichung mittels Inhalation geeignete Rezeptursuspension erhalten wird, worin die ursprüngliche Teilchengrößen-Verteilung des Wasser-unlöslichen aktiven Prinzips beibehalten wird.

2. Brausetablette nach Anspruch 1, worin das aktive Prinzip ausgewählt ist unter Corticosteroiden und mucolytischen Mitteln.

3. Brausetablette nach Anspruch 1 oder 2, worin das aktive Prinzip ausgewählt ist aus der Gruppe bestehend aus Beclomethasondipropionat, Fluticasonpropionat, Budesonid, Flunisolid, Betamethason, Triamcinolon, Mometason, Ciclesonid und Sobrerol.

4. Brausetablette nach Anspruchs 1 bis 3, worin das aktive Prinzip ausgewählt ist unter Beclomethasonedipropionat und Fluticasonepropionat.

5. Verwendung einer Brausetablette nach Anspruchs 1 bis 4 zur Zubereitung einer Rezeptursuspension zur Verabreichung mittels Inhalation.

6. Verwendung nach Anspruch 5, welches den Schritt der Desintegration der Brausetablette in Wasser oder in einer wäßrigen Lösung umfasst.

7. Verwendung nach Anspruch 6, wobei die wäßrige Lösung eine physiologische Lösung ist.

8. Verwendung nach Anspruch 6 bis 7, wobei die wäßrige Lösung eine Lösung von mindestens einem Wasser-löslichen aktiven Prinzip ist.

9. Verwendung nach Anspruch 8, worin das Wasser-lösliche aktive Prinzip ein mukolytisches Mittel oder ein Bronchiodilator ist.

10. Verwendung nach Anspruch 9, wobei das mukolytische Mittel ausgewählt ist aus der Gruppe bestehend aus Ambroxol oder Sobrerol.

11. Verwendung nach Anspruch 9, wobei der Bronchodilator Salbutamol oder Ipratropiumchlorid ist.

## Revendications

1. Comprimé effervescent destiné à la préparation extemporanée d'une suspension adaptée à une administration par inhalation comprenant au moins un principe actif insoluble dans l'eau et des excipients adaptés à une administration par inhalation comprenant :
un composé acide choisi parmi l'acide citrique, l'acide tartrique,
un composé basique choisi parmi un carbonate, un bicarbonate, un carbonate cuit et un bicarbonate cuit ;
des tensioactifs non ioniques ou ioniques,
dans lequel ledit principe actif insoluble dans l'eau a été micronisé à une granulométrie située entre 0,5 et 7 µm et
lequel comprimé effervescent, une fois ajouté à l'eau ou à une solution aqueuse, se désagrège jusqu'à obtention de ladite suspension extemporanée adaptée à une administration par inhalation, dans laquelle la distribution granulométrique initiale dudit principe actif insoluble dans l'eau est maintenue.

2. Comprimé effervescent selon la revendication 1, dans lequel ledit principe actif est choisi parmi les corticostéroïdes et les agents mucolytiques.

3. Comprimé effervescent selon les revendications 1 ou 2, dans lequel ledit principe actif est choisi dans le groupe constitué du dipropionate de béclométhasone, du propionate de fluticasone, du budésonide, du flunisolide, de la bêtaméthasone, de la triamcinolone, de la mométasone, du ciclésonide et du sobrerol.

4. Comprimé effervescent selon les revendications 1 à 3 dans lequel ledit principe actif est choisi parmi le dipropionate de béclométhasone et le propionate de fluticasone.

5. Utilisation d'un comprimé effervescent selon les revendications 1 à 4 pour la préparation d'une suspension extemporanée destinée à une administration par inhalation.

6. Utilisation selon la revendication 5, comprenant l'étape de désintégration du comprimé effervescent dans l'eau ou dans une solution aqueuse.

7. Utilisation selon la revendication 6, dans laquelle ladite solution aqueuse est une solution physiologique.

8. Utilisation selon les revendications 6 à 7, dans laquelle ladite solution aqueuse est une solution d'au moins un principe actif soluble dans l'eau.

9. Utilisation selon la revendication 8, dans laquelle ledit principe actif soluble dans l'eau est un agent mucolytique ou un bronchodilatateur.

10. Utilisation selon la revendication 9, dans laquelle ledit agent mucolytique est choisi dans le groupe constitué de l'ambroxol ou du sobrerol.

11. Utilisation selon la revendication 9, dans laquelle ledit bronchodilatateur est du salbutamol ou du chlorure d'ipratropium.
